# EUROPÄISCHE PATENTSCHRIFT

(11) **EP 1 315 481 B1**
(45) Veröffentlichungstag und Bekanntmachung des Hinweises auf die Patenterteilung: **06.12.2006**
(21) Anmeldenummer: 00952830.8
(22) Anmeldetag: 29.08.2000
(51) Int. Cl.: A61K 9/52

(54) **ARZNEIMITTEL ZUR BEHANDLUNG VON DARMERKRANKUNGEN**
MEDICAMENT FOR TREATING INTESTINAL DISEASES
MEDICAMENT POUR LE TRAITEMENT DE MALADIES INTESTINALES

(43) Veröffentlichungstag der Anmeldung: 04.06.2003
(73) Patentinhaber: Mepha AG, CH-4147 Aesch (CH)
(72) Erfinder: LÜTOLF, Peter, CH-4127 Birsfelden (CH); MAURER, Reto, CH-4053 Basel (CH); SCHEIWE, Werner, 79689 Maulburg (DE)
(74) Vertreter: Braun, André jr.
(86) Internationale Anmeldenummer: PCT/CH2000/000457
(87) Internationale Veröffentlichungsnummer: WO 2002/017887

(56) Entgegenhaltungen:
- EP-A- 0 943 341
- DE-A- 19 732 903
- M. Z. I. KHAN ET AL.: "a pH-dependent colon-targeted oral drug delivery system using methacrylic acid copolymers. II. manipulation of drug release using Eudragit L100 and Eudragit S100 combinations" DRUG DEVELOPMENT AND INDUSTRIAL PHARMACY, Bd. 25, Nr. 05, Mai 2000 (2000-05), Seiten 549-554, XP000981442 New York (US)

## Beschreibung

Die vorliegende Erfindung betrifft ein Arzneimittel zur oralen Verabreichung, aus welchem der Wirkstoff vorwiegend in den unteren Darmabschnitten freigesetzt wird.

Als Wirkstoff kann das erfindungsgemässe Arzneimittel 5-Aminosalicylsäure oder ein pharmazeutisch annehmbares Salz oder einen pharmazeutisch annehmbaren Ester davon enthalten oder auch ein Glucocorticoid.

Das erfindungsgemässe Arzneimittel eignet sich zur lokalen Behandlung von Darmerkrankungen. Es besteht aus den Wirkstoff enthaltenden Pellets, Granulaten oder Minitabletten konventioneller Natur, welche einzeln überzogen sind mit einem magensaftresistenten Lack, enthaltend ein Gemisch eines ersten anionischen Copolymeren von Methacrylsäure und Methacrylsäuremethylester, worin das Verhältnis zwischen freien Carboxylgruppen und Methylestergruppen etwa 1:1 beträgt, mit einem zweiten anionischen Copolymeren von Methacrylsäure und Methacrylsäuremethylester, worin das Verhältnis zwischen freien Carboxylgruppen und Methylestergruppen etwa 1:2 beträgt, wobei das Verhältnis des ersten zum zweiten Copolymeren etwa 1:2 bis etwa 1:9 ist und wobei die Menge des aufgetragenen Lacks mindestens etwa 30%, bezogen auf die unbeschichteten Pellets bzw. Granulate bzw. Minitabletten, ist.

Die unbeschichteten Pellets oder Granulate haben einen Durchmesser von etwa 0.1-2.5 mm, vorzugsweise etwa 0.7 mm, und die unbeschichteten Minitabletten haben einen Durchmesser von etwa 1-5 mm. Die Menge des Lacks, bezogen auf die unbeschichteten Pellets bzw. Granulate bzw. Minitabletten, beträgt vorzugsweise etwa 35-55%.

Die beschichteten Pellets bzw. Granulate bzw. Minitabletten können zusammen mit einem Pulvergemisch aus einem oder mehreren Zuckeralkoholen und einer Säure sowie gegebenenfalls einem oder mehreren Quellmittel(n) und/oder Aromastoff(en) in Sachets oder andere geeignete Behälter eingefüllt werden, deren Inhalt daraus entnommen und in trockenem Zustand oder nach Vordispergieren in wenig Wasser eingenommen werden kann.

Die 5-Aminosalicylsäure oder pharmazeutisch annehmbare Salze oder Ester davon werden zur Behandlung entzündlicher Darmerkrankungen, wie Colitis ulcerosa oder Morbus Crohn, eingesetzt. Ihre Wirksamkeit beruht vor allem auf lokalen Effekten im Ileum bzw. Kolon; die systemische Verfügbarkeit ist indessen unerwünscht. Bei konventionellen oralen Verabreichungsformen, welche den Wirkstoff im Magen oder im Duodenum freisetzen, würde ein verminderter therapeutischer Effekt auf die entzündlichen Darmerkrankungen erzielt, da der freigesetzte Wirkstoff fast vollständig im oberen Dünndarm resorbiert und damit nicht in ausreichender Menge die weiter unten lokalisierten Darmabschnitte erreichen würde.

Auf dem Markt existieren bereits mit magensaftresistentem Lack überzogene Tabletten zu 250, 400 und 500 mg 5-Aminosalicylsäure (Salofalk®, Asacol®, Claversal®). Ebenfalls auf dem Markt erhältlich sind mit Ethylcellulose retardierte Darreichungsformen der 5-Aminosalicylsäure, nämlich Tabletten zu 250 und 500 mg (Pentasa®) sowie neuerdings auch ein Granulat in Portionen von 1g (Pentasa® retard); da die Freisetzung aus diesen mit Ethylcellulose retardierten Formen pH-unabhängig ist, wird der Wirkstoff bereits im Magen und Dünndarm freigesetzt, was jedoch, wie oben erwähnt, unerwünscht ist.

In der Literatur, insbesondere in der Patentliteratur, sind zahlreiche verschiedene, jeweils durch bestimmte Merkmale gekennzeichnete Darreichungsformen für die 5-Aminosalicylsäure und z.T. für deren pharmazeutisch annehmbaren Salze und Ester beschrieben worden.

So wird in EP 0 083 775 B2 ein 5-Aminosalicylsäure enthaltendes Arzneimittel beschrieben, bei dem sich keine unvertretbaren Stabilitätsprobleme ergeben. Dieses Arzneimeittel enthält ein Trockengemisch aus (a) 5-Aminosalicylsäure und (b) einem basischen Hilfsstoff und/oder einem damit vermengten Puffergemisch, wobei Komponente (b) in 1%iger wässriger Lösung pH-Werte zwischen 8 und 12 ergibt und wobei die Komponenten (a) und (b) nicht durch einen Überzug aus Polyvinylpyrrolidon getrennt sind. Als Formen für dieses Arzneimittel werden genannt: Suppositorien; Granulate mit mittlerer Korngrösse 0.15-0.5 und 0.6-1.5 mm zwecks Herstellung von Tabletten, Dragées oder Kapseln, erhalten durch Brechen entsprechender Briketts; Tabletten, Filmtabletten, Dragées, Kapseln oder Granulate mittlerer Korngrösse >1.5 mm, welche in an sich bekannter Weise magensaftresistent umhüllt sind, z.B. mit Celluloseacetatphthalat oder mit dem als Eudragit® S bekannten anionischen Polymerisat aus Methacrylsäure und Methacrylsäuremethylester mit einem Verhältnis zwischen freien Carboxylgruppen und Methylestergruppen von etwa 1:2 und einem mittleren Molekulargewicht von etwa 135'000.

In EP 0 097 651 B1 werden oral verabreichbare Zusammensetzungen für die selektive Freisetzung von 5-Aminosalicylsäure oder einem pharmazeutisch annehmbaren Salz oder Ester davon im Dickdarm beschrieben. Bei diesen Zubereitungen handelt es sich um den Wirkstoff enthaltende feste orale Dosierungsformen, wie Kapseln oder Tabletten, welche mit einer 60-150 µm dicken Schicht eines anionischen Polymeren beschichtet sind, das im Magensaft und im Darmsaft unterhalb eines pH-Wertes von 7 unlöslich, im Kolonsaft jedoch ausreichend löslich ist, um 5-Aminosalicylsäure im Dickdarm abzugeben. Als besonders bevorzugtes anionisches Polymer wird Eudragit® S erwähnt.

WO 97/23199 A1 beschreibt oral verabreichbare Zusammensetzungen zur Behandlung entzündlicher Darmerkrankungen mit Freisetzung von 5-Aminosalicylsäure im Dünndarm und im Dickdarm in Form einzeln beschichteter Granula, jeweils bestehend aus (a) einem Kern enthaltend 5-Aminosalicylsäure oder ein pharmazeutisch annehmbares Salz oder einen pharmazeutisch annehmbaren Ester davon und einen physiologisch annehmbaren ersten Hilfsstoff, vorzugsweise ein Cellulosederivat, insbesondere mikrokristalline Cellulose, und (b) einen zweiten Hilfsstoff als Überzug, vorzugsweise ein semipermeables Polymer, insbesondere Ethylcellulose; diese Granula sind mehrheitlich weitgehend sphärisch und haben mehrheitlich einen Durchmesser von 0.5-2.0 mm, vorzugsweise 0.7-1.1 mm.

WO 98/22096 A1 beschreibt oral verabreichbare Zubereitungen zwecks Freisetzung verschiedener therapeutischer Wirkstoffe, wie u.a. 5-Aminosalicylsäure, im oder nahe beim Eingang des Kolons. Diese Zubereitungen umfassen (a) eine effektive Menge des therapeutischen Wirkstoffs in oder auf der Oberfläche einer Dosierungsform ausgewählt aus einem sphärischen Substrat, einem elliptischen Substrat, einer Hartkapsel oder einer komprimierten Tablette, deren grösste Abmessung etwa 3-10 mm ist, vorzugsweise eine Kugel mit einem Durchmesser von etwa 3-8 mm, und (b) einen magensaftresistenten Überzug, bestehend aus mindestens einer inneren und mindestens einer äusseren Schicht, wobei die innere(n) Schicht(en) ein magensaftresistentes Polymer ist (sind), das sich in wässrigem Medium bei einem pH von etwa 5-6.3 aufzulösen beginnt, und die äussere(n) Schicht(en) ein magensaftresistentes Polymer ist (sind), das sich in wässrigem Medium bei einem pH von etwa 6.8-7.2 aufzulösen beginnt. Als für die innere(n) Schicht(en) geeignete Polymere werden genannt: Celluloseacetatphthalat, Celluloseacetattrimelliat, Hydroxypropylmethylcellulosephthalat, Hydroxypropylmethylcelluloseacetatsuccinat, Polivinylacetetphthalat sowie Copolymerisate von Methacrylsäure und Methacrylsäuremethylester oder Acrylsäureethylester, wie das Produkt Eudragit® L, ein anionisches Copolymerisat von Methacrylsäure und Methacrylsäuremethylester mit einem Verhältnis zwischen freien Carboxylgruppen und Methylestergruppen von etwa 1:1 und einem mittleren Molekulargewicht von etwa 135'000. Als für die äussere(n) Schicht(en) geeignete Polymere werden Copolymere von Methacrylsäure und Methacrylsäuremethylester genannt, wie Eudragit® S und auch Mischungen davon mit Eudragit® L.

In M.Z.I. Khan et. al, Drug Development and Industrial Pharmacy, 26(5), 549-554 (2000) werden 125 mg 5-Aminosalicylsäure enthaltende Tabletten beschrieben, welche mit Kombinationen von Eudragit® L 100 und Eudragit® S 100 in verschiedenen Mengenverhältnissen überzogen sind; wobei als bevorzugtes Verhältnis 4:1 angegeben wird (Seite 551, linke Spalte, letzte Zeile); demgegenüber beträgt bei den erfindungsgemässen Arzneimitteln das Verhältnis von Copolymeren wie Eudragit® L 100 zu Copolymeren wie Eudragit® S 100 etwa 1:2 bis etwa 1:9.

Ziel der vorliegenden Erfindung ist es, ein oral zu verabreichendes Arzneimittel bereitzustellen, welches
- in einer Dosierungseinheit präsentiert werden kann, welche auch eine sehr hohe Einzeldosis von etwa 1-2 g des Wirkstoffs, wie 5-Aminosalicylsäure, enthalten kann;
- magensaftresistent ist ( in vitro keine Freisetzung des Wirkstoffs in 0.1 N HCl);
- den Wirkstoff erst ab dem terminalen Ileum abgibt (in vitro möglichst geringe Freisetzung bei pH 6.5 während etwa 100 min);
- stabil ist, indem keine Oxidation des Wirkstoffs eintritt; und
- angenehm einzunehmen ist.

Bisher existierte kein orales Arzneimittel, welches pro Dosierungseinheit 5-Aminosalicylsäure in einer derartigen Menge enthält, nämlich in der Grössenordnung von 1.5 g; die Entwicklung einer derart hoch dosierten oralen Darreichungsform von 5-Aminosalicylsäure stellte offenbar ein Problem dar.

Da die Tagesdosis in der akuten Krankheitsphase 4.5 g betragen kann, stellt die erfindungsgemäss erzielbare Dosierungseinheit von 1.5 g insofern einen erheblichen Vorteil dar, als pro Tag anstelle von beispielsweise 18 Tabletten zu 250 mg oder 9 Tabletten zu 500 mg lediglich drei erfindungsgemässe Dosierungseinheiten (wie Sachets) zu 1.5 g eingenommen werden müssen, was natürlich die Befolgung der verordneten Therapie durch den Patienten ("patient compliance") verbessert. Auch kann das erfindungsgemässe Arzneimittel leicht und bequem eingenommen werden: Der die beschichteten Pellets bzw. Granulate bzw. Minitabletten enthaltende Inhalt der Sachets kann in trockenem Zustand eingenommen werden, z.B. mit einem Löffel, oder nach Vordispergieren in wenig Wasser.

Werden Pellets üblicher Grösse mit einem Durchmesser ≥ 1 mm oral eingenommen, dann bleiben diese, bei Vordispergieren in einem Glas Wasser, teilweise im Glas zurück, und sie kleben auch zwischen den Zähnen. Die erfindungsgemässen Pellets bzw. Granulate sind jedoch kleiner, indem ihr Durchmesser vorzugsweise etwa 0.7 mm beträgt; zudem werden diese Pellets bzw. Granulate bzw. die erfindungsgemässen Minitabletten zweckmässigerweise zusammen mit einem Pulvergemisch aus einem oder mehreren Zuckeralkoholen (dichteerhöhend beim Vordispergieren in Wasser) und einer Säure (pH-Korrektur, damit der Wirkstoff nicht bereits beim Vordispergieren oder im Speichel freigesetzt wird) sowie gegebenenfalls mit einem oder mehreren Quellmitteln und/oder Aromastoffen in Sachets oder andere geeignete Behälter eingefüllt. All dies verbessert die Schluckbarkeit und den Einnahmekomfort.

Die erfindungsgemässen Arzneimittel weisen auch eine höhere Sicherheit auf als, mit einem magensaftresistenten Film beschichtete Tabletten oder Kapseln, denn eine unsachgemässe Einnahme, nämlich Zerbeissen der Arzneiform, ist bei Pellets, Granulaten und Minitabletten wesentlich weniger kritisch.

Wie eingangs erwähnt enthält der auf die Pellets bzw. Granulate bzw. Minitabletten konventioneller Natur aufgebrachte Lack ein Gemisch eines ersten anionischen Copolymeren von Methacrylsäure und Methacrylsäuremethylester, worin das Verhältnis zwischen freien Carboxylgruppen und Methylestergruppen etwa 1:1 beträgt, mit einem zweiten anionischen Copolymeren von Methacrylsäure und Methacrylsäuremethylester, worin das Verhältnis zwischen freien Carboxylgruppen und Methylestergruppen etwa 1:2 beträgt. Das Mengenverhältnis zwischen dem ersten und dem zweiten Copolymeren beträgt etwa 1:2 bis etwa 1:9, vorzugsweise etwa 1:3. Sowohl für das erste als auch für das zweite Copolymere liegt das mittlere Molekulargewicht zweckmässigerweise in der Grössenordnung von 135'000. Besonders geeignete als erstes bzw. als zweites Copolymer verwendbare Produkte sind im Handel unter der Bezeichnung Eudragit® L bzw. Eudragit® S erhältlich.

Ausser den erwähnten Copolymeren enthält der Lack zweckmässigerweise einen Weichmacher und ein Antiklebmittel. Dabei können etwa 10-80% Weichmacher und 10-80% Antiklebmittel, jeweils bezogen auf die Polymer-Trockenmasse zugesetzt werden.

Als Weichmacher eignen sich Triethylcitrat, Dibutylsebacat, Propylenglycole, Dibutylphthalat, Diethylphthalat, Tributylcitrat, Triacetin, oder Ricinusöl; ein besonders geeigneter Weichmacher ist Triethylcitrat (Citroflex®-2).

Geeignete Antiklebmittel sind beispielsweise Talk oder amorphe Kieselsäure.

Als Zuckeralkohole für das zusammen mit den beschichteten Pellets, Granulaten oder Minitabletten in Sachets oder in andere geeignete Behälter einzufüllende Pulvergemisch eignen sich beispielsweise Mannitol, Sorbitol oder Xylitol. Als Säure eignet sich beispielsweise Citronensäure. Als Quellmittel kann man beispielsweise mikrokristalline Cellulose oder ein Cellulosederivat verwenden.

Die erfindungsgemässen Arzneimittel lassen sich nach an sich bekannten und in der Fachwelt allgemein üblichen Methoden herstellen.

So wird beispielsweise zur Herstellung der unbeschichteten Pellets der Wirkstoff zusammen mit üblichen Bindemitteln, wie Hydroxypropylmethylcellulose, Polyvinylpyrrolidon oder Maisstärke, einem Säuerungsmittel, wie Citronensäure, und gegebenenfalls Füllstoffen, wie mikrokristalline Cellulose oder Lactose, und/oder Antioxidantien, wie Butylhydroxyanisol oder Ascorbinsäure, vermischt, worauf das Gemisch geknetet und dann durch eine Lochscheibe mit Löchern geeigneter Grösse (z.B. 0.7 mm) extrudiert wird; danach wird spheronisiert und getrocknet.

Die unbeschichteten Pellets bzw. Granulate bzw. Minitabletten werden in üblicher Weise mit dem Lack versehen, wobei der Lack ausser den Copolymeren, Weichmachern und Antiklebmitteln auch noch geeignete Pigmente, wie z.B. das Weisspigment Titandioxid, enthalten kann. Nach beendetem Lackauftrag werden die lackierten Pellets bzw. Granulate bzw. Minitabletten getrocknet.

Schliesslich können die mit dem Lack beschichteten Pellets bzw. Granulate bzw. Minitabletten zusammen mit der der Verbesserung und Erleichterung der Einnahme dienenden Pulvermischung in Sachets geeigneter Grösse oder in andere geeignete Behälter entsprechender Grösse abgefüllt werden.

Die nachfolgenden Beispiele sollen die Erfindung erläutern, ihren Umfang aber in keiner Weise einschränken.

### Beispiel 1

Nach allgemein üblichen Methoden wurden unbeschichtete Pellets hergestellt aus

| | |
|---|---|
| 5-Aminosalicylsäure | 1'500.0 mg |
| mikrokristalline Cellulose | 381.5 mg |
| Lactose | 57.3 mg |
| Citronensäure | 2.3 mg |
| Ascorbinsäure | 1.0 mg |
| Hydroxypropylmethylcellulose | 52.5 mg |
| | |
| Total | 1'994.6 mg |

### Beispiel 2 (Vergleich)

Nach allgemein üblichen Methoden wurden mit Lack beschichtet Pellets hergestellt aus

| | |
|---|---|
| Pellets aus Beispiel 1 | 1'994.6 mg |
| Eudragit® L 100 | 79.5 mg |
| Eudragit® S 100 | 238.4 mg |
| Citroflex®-2 | 158.9 mg |
| Talk | 79.5 mg |
| Total | 2'550.8 mg |

### Beispiel 3

Nach allgemein üblichen Methoden wurden mit Lack beschichtet Pellets hergestellt aus

| | |
|---|---|
| Pellets aus Beispiel 1 | 1'994.6 mg |
| Eudragit® L 100 | 119.2 mg |
| Eudragit® S 100 | 357.6 mg |
| Citroflex®-2 | 238.4 mg |
| Talk | 119.2 mg |
| | |
| Total | 2'828.0 mg |

### Beispiel 4

Nach allgemein üblichen Methoden wurden mit Lack beschichtet Pellets hergestellt aus

| | |
|---|---|
| Pellets aus Beispiel 1 | 1'994.6 mg |
| Eudragit® L 100 | 89.2 mg |
| Eudragit® S 100 | 267.5 mg |
| Citroflex®-2 | 178.3 mg |
| Talk | 89.2 mg |
| Titandioxid | 85.0 mg |
| | |
| Total | 2'703.8 mg |

### Beispiel 5

Es wurde eine Pulvermischung hergestellt aus

| | |
|---|---|
| Beschichtete Pellets aus Beispiel | 3 2'828.0 mg |
| Citronensäure | 73.0 mg |
| Mikrokristalline Cellulose | 750.0 mg |
| Mannitol | 1'490.0 mg |
| Sorbitol | 2'720.0 mg |
| Orangenaroma | 40.0 mg |
| | |
| Total | 7'902.0 mg |

Diese Pulvermischung, enthaltend 1.5 g 5-Aminosalicylsäure, kann in ein Sachet geeigneter Grösse abgefüllt werden.

### Beispiel 6

Die Freisetzung von 5-Aminosalicylsäure aus den beschichteten Pellets gemäss Beispielen 2, 3 und 4 wurde in vitro unter folgenden Bedingungen ermittelt: Bio-Dis (USP-Apparatur Nr. 3, nur 100 mg 5-Aminosalicylsäure pro Probe, da sonst Sink-Bedingungen nicht eingehalten werden können), 37°C, 2h in 200 ml pH 1.2; 2h in 200 ml USP Puffer pH 6.5; 1h in 200 ml USP Puffer pH 7.2; nochmals 1h in 200 ml USP Puffer pH 7.2; 20 Dips pro Minute; 405 µm Polypropylen-Sieb; Analyse der freigesetzten 5-Aminosalicylsäure mittels validierter HPLC-Methode.

Die befilmten Pellets gemäss den Beispielen 2, 3 und 4 waren während 2 Stunden magensaftresistent. Die Pellets gemäss Beispiel 2 mit 28% Lack bezogen auf unbeschichtete Pellets setzten in den nachfolgenden 2 Stunden bei pH 6.5 im Mittel 19% 5-Aminosalicylsäure frei, diejenigen gemäss Beispiel 3 mit 42% Lack bezogen auf unbeschichtete Pellets 7% und diejenigen gemäss Beispiel 4 mit 36% Lack bezogen auf unbeschichtete Pellets 6%. Das Anforderungsprofil bezüglich in-vitro-Freisetzung wird von den Pellets gemäss Beispielen 3 und 4 erfüllt, von denjenigen gemäss Beispiel 2 jedoch nicht vollumfänglich; die erfindungsgemässen Arzneimittel enthalten daher mindestens etwa 30%, vorzugsweise mindestens etwa 35% Lack bezogen auf unbeschichtete Pellets bzw. Granulate bzw. Minitabletten.

## Patentansprüche

1. Arzneimittel zur oralen Verabreichung, aus welchem der Wirkstoff vorwiegend in den unteren Darmabschnitten freigesetzt wird, bestehend aus den Wirkstoff enthaltenden Pellets, Granulaten oder Minitabletten konventioneller Natur, welche einzeln überzogen sind mit einem magensaftresistenten Lack, enthaltend ein Gemisch eines ersten anionischen Copolymeren von Methacrylsäure und Methacrylsäuremethylester, worin das Verhältnis zwischen freien Carboxylgruppen und Methylestergruppen etwa 1:1 beträgt, mit einem zweiten anionischen Copolymeren von Methacrylsäure und Methacrylsäuremethylester, worin das Verhältnis zwischen freien Carboxylgruppen und Methylestergruppen etwa 1:2 beträgt, wobei das Verhältnis des ersten zum zweiten Copolymeren etwa 1:2 bis etwa 1:9 ist und wobei die Menge des aufgetragenen Lacks mindestens etwa 30%, bezogen auf die unbeschichteten Pellets bzw. Granulate bzw. Minitabletten, ist.

2. Arzneimittel gemäss Anspruch 1, enthaltend als Wirkstoff 5-Aminosalicylsäure oder ein pharmazeutisch annehmbares Salz oder einen pharmazeutisch annehmbaren Ester davon oder ein Glucocorticoid.

3. Arzneimittel gemäss Anspruch 1 oder 2, worin die unbeschichteten Pellets oder Granulate einen Durchmesser von etwa 0.1-2.5 mm bzw. die unbeschichteten Minitabletten einen Durchmesser von etwa 1-5 mm aufweisen.

4. Arzneimittel gemäss einem der Ansprüche 1 bis 3, worin die Menge des Lacks, bezogen auf die unbeschichteten Pellets bzw. Granulate bzw Minitabletten, etwa 35-55% beträgt.

5. Arzneimittel gemäss einem der Ansprüche 1 bis 4, worin die anionischen Copolymeren von Methacrylsäure und Methacrylsäuremethylester ein mittleres Molekulargewicht in der Grössenordnung von 135'000 aufweisen.

6. Arzneimittel gemäss einem der Ansprüche 1 bis 5, worin das Verhältnis des ersten zum zweiten Copolymeren etwa 1:3 ist.

7. Arzneimittel gemäss einem der Ansprüche 1 bis 6, worin etwa 10-80% Weichmacher und etwa 10-80% Antiklebmittel, beides bezogen auf die Polymer-Trockenmasse, im Lack enthalten sind.

8. Arzneimittel gemäss einem der Ansprüche 1 bis 7, worin die beschichteten Pellets bzw. Granulate bzw. Minitabletten zusammen mit einem Pulvergemisch aus einem oder mehreren Zuckeralkoholen und einer Säure sowie gegebenenfalls einem oder mehreren Quellmittel(n) und/oder Aromastoff(en) in Sachets oder andere geeignete Behälter eingefüllt werden, deren Inhalt daraus entnommen und in trockenem Zustand oder nach Vordispergieren in wenig Wasser eingenommen werden kann.

9. Arzneimittel gemäss Anspruch 8, welches als Zuckeralkohol(e) Mannitol und/oder Sorbitol und als Säure Citronensäure enthält, wobei es als fäkultative(s) Quellmittel mikrokristalline Cellulose und/oder ein Cellulosederivat enthalten kann.

10. Arzneimittel gemäss Anspruch 8 oder 9, worin die in einem Sachet oder einem anderen geeigneten Behälter vorhandene Menge etwa 1-2 g, vorzugsweise etwa 1.5 g, Wirkstoff entspricht.

## Claims

1. Drug for oral administration from which the active ingredient is released predominantly in the lower sections of the intestine, consisting of pellets, granules or minitablets of a conventional nature containing the active ingredient, which are individually coated with an enteric lacquer containing a mixture of a first anionic copolymer of methacrylic acid and methyl methacrylate wherein the ratio of free carboxyl groups to methyl ester groups is about 1:1, and with a second anionic copolymer of methacrylic acid and methyl methacrylate wherein the ratio of free carboxyl groups to methyl ester groups is about 1:2, the ratio of the first copolymer to the second being about 1:2 to about 1:9 and the amount of lacquer applied being at least about 30%, based on the uncoated pellets, granules or minitablets.

2. Drug according to Claim 1 in which the active ingredient is 5-aminosalicylic acid or a pharmaceutically acceptable salt or a pharmaceutically acceptable ester thereof or a glucocorticoid.

3. Drug according to Claim 1 or 2 wherein the uncoated pellets or granules have a diameter of about 0.1 - 2.5 mm or the uncoated minitablets have a diameter of about 1 - 5 mm.

4. Drug according to one of Claims 1 to 3 wherein the amount of lacquer is about 35 - 55%, based on the uncoated pellets, granules or minitablets.

5. Drug according to one of Claims 1 to 4 wherein the anionic copolymers of methacrylic acid and methyl methacrylate have an average molecular weight in the order of 135,000.

6. Drug according to one of Claims 1 to 5 wherein the ratio of the first copolymer to the second is about 1:3.

7. Drug according to one of Claims 1 to 6 wherein the lacquer contains about 10 - 80% of plasticizer and about 10 - 80% of anticaking agent, both based on the dry weight of polymer.

8. Drug according to one of Claims 1 to 7 wherein the coated pellets, granules or minitablets are introduced into sachets or other suitable containers together with a pulverulent mixture of one or more sugar alcohols and an acid and optionally one or more swelling agents and/or flavourings, it being possible for the contents of said sachets or other suitable containers to be removed therefrom and taken in the dry state or after dispersion in a small amount of water.

9. Drug according to Claim 8 in which the sugar alcohol(s) is (are) mannitol and/or sorbitol and the acid is citric acid, it being possible for said drug to contain microcrystalline cellulose and/or a cellulose derivative as the optional swelling agent(s).

10. Drug according to Claim 8 or 9 wherein the amount present in a sachet or other suitable container corresponds to about 1 - 2 g, preferably about 1.5 g, of active ingredient.

## Revendications

1. Un médicament pour l'administration par voie orale duquel la substance active est essentiellement libérée dans les parties inférieures de l'intestin, composé de pellets, granulés ou mini-comprimés de nature conventionnelle qui comprennent la substance active, qui sont enrobés individuellement avec un vernis résistant au suc gastrique, comprenant un mélange d'un premier copolymère anionique d'acide méthacrylique et d'ester méthylique de l'acide méthacrylique, où la proportion entre les groupes carboxyles libres et les groupes d'ester méthylique est d'environ 1:1, avec un deuxième copolymère anionique d'acide méthacrylique et d'ester méthylique de l'acide méthacrylique, où la proportion entre les groupes carboxyles et les groupes d'ester méthylique est d'environ 1:2, où la proportion du premier copolymère par rapport au deuxième est d'environ 1:2 à environ 1:9 et où la quantité du vernis appliqué est d'au moins environ 30 %, calculée par rapport aux pellets resp. granulés resp. mini-comprimés non enrobés.

2. Un médicament selon la revendication 1, comprenant en tant que substance active de l'acide 5-aminosalicylique ou un sel pharmaceutiquement acceptable ou un ester pharmaceutiquement acceptable de celui-ci ou un glycocorticoïde.

3. Un médicament selon les revendications 1 ou 2, où les pellets ou les granulés non enrobés ont un diamètre d'environ 0.1 - 2.5 mm resp. où les mini-comprimés non enrobés ont un diamètre d'environ 1 - 5 mm.

4. Un médicament selon l'une quelconque des revendications 1 à 3, où la quantité de vernis s'élève à environ 35 - 55 %, calculée par rapport aux pellets resp. granulés resp. mini-comprimés non enrobés.

5. Un médicament selon l'une quelconque des revendications 1 à 4, où les copolymères anioniques d'acide méthacrylique et d'ester méthylique de l'acide méthacrylique ont un poids moléculaire moyen de l'ordre de 135000.

6. Un médicament selon l'une quelconque des revendications 1 à 5, où la proportion du premier copolymère par rapport au deuxième est d'environ 1:3.

7. Un médicament selon l'une quelconque des revendications 1 à 6, où environ 10 - 80 % d'agent adoucissant et environ 10 - 80 % d'agent antiadhésif, les deux calculés par rapport à la matière sèche de polymère, sont compris dans le vernis.

8. Un médicament selon l'une quelconque des revendications 1 à 7, où les pellets resp. les granulés resp. les mini-comprimés enrobés ensemble avec un mélange poudreux à partir d'un ou de plusieurs sucre-alcools et d'un acide ainsi qu'optionnellement avec un ou plusieurs agents gonflants et/ ou aromatisants sont remplis dans des sachets ou d'autres récipients adaptés, d'où le contenu peut être prélevé et pris à l'état sec ou après prédispersion dans un peu d'eau.

9. Un médicament selon la revendication 8, qui comprend en tant que sucre-alcool(s) du mannitol et/ ou du sorbitol et en tant qu'acide de l'acide citrique, où en tant qu'agent(s) gonflant(s) facultatif(s) peut être contenu de la cellulose microcristalline et/ ou un dérivé de cellulose.

10. Un médicament selon les revendications 8 ou 9, où la quantité présente dans un sachet ou dans un autre récipient adapté correspond à environ 1 - 2 g, de préférence environ 1.5 g de substance active.
